Europäisches Patentamt

European Patent Office

Office européen des brevets

(11) Publication number: **0 281 186 B1**

# EUROPEAN PATENT SPECIFICATION

(45) Date of publication of patent specification: **28.10.92**  (51) Int. Cl.⁵: **C07C 233**/64, C07C 63/70, C07C 231/00, C07C 51/58

(21) Application number: **88200301.5**

(22) Date of filing: **18.02.88**

(54) Process for the preparation of a benzoic acid derivative.

(30) Priority: **05.03.87 GB 8705187**

(43) Date of publication of application:
**07.09.88 Bulletin 88/36**

(45) Publication of the grant of the patent:
**28.10.92 Bulletin 92/44**

(84) Designated Contracting States:
**AT BE CH DE ES FR GB GR IT LI LU NL SE**

(56) References cited:
**EP-A- 0 140 482**

**PATENT ABSTRACTS OF JAPAN, unexamin-
ed applications, C field, vol. 9, no. 287, No-
vember 14, 1985; THE PATENT OFFICE JAPA-
NESE GOVERMENT, page 130 C 314**

**CHEMICAL ABSTRACTS, vol. 66, no. 21, May
22, 1967, Columbus, Ohio, USA; V. M.
VLASOV et al, "Aromatic fluoro derivatives.
XXV. Fluorine-containing benzoic acids",
page 8855, column 2, abstract no. 94 742f**

(73) Proprietor: **SHELL INTERNATIONALE RE-
SEARCH MAATSCHAPPIJ B.V.
Carel van Bylandtlaan 30
NL-2596 HR Den Haag(NL)**

(72) Inventor: **Prillwitz, Peter Ernst
Badhuisweg 3
NL-1031 CM Amsterdam(NL)**
Inventor: **Heijmen, Hendricus Joannes
Badhuisweg 3
NL-1031 CM Amsterdam(NL)**

## Description

This invention relates to a process for the preparation of a benzoic acid derivative.

2,6-Difluorobenzamide is a key intermediate in the preparation of several acyl urea insecticides; see for example UK Specifications 1324293 and 1460419. In recent years, much research has been devoted towards finding new and improved routes for preparing the compound. While many routes have been suggested, the commercial production of large quantities of material remains difficult and expensive.

2,6-Difluorobenzamide can only be prepared from cheap and readily available starting materials in a number of steps. The introduction of the fluorine atom into the benzene ring takes up at least one of those steps. Since the introduction of the fluorine atom is difficult and expensive to perform, it should desirably be effected at a stage as late as possible in the preparative route. In that way, the cost associated with the loss of material which occurs during each stage of the synthesis is minimised.

Many ways are known for introducing fluorine atoms into benzene rings. For example they may be introduced by heating a diazonium fluoroborate, which itself may be prepared from the corresponding aniline. Alternatively, they may, in certain cases, be prepared from another halobenzene by a halogen exchange reaction.

The applicants are not aware of any published method for preparing 2,6-difluorobenzamide which involves the final stage introduction of a fluorine atom into the benzene ring. However, a method which involves fluorination in the penultimate stage is known. This method comprises reacting 2,6-dichlorobenzonitrile with an alkali metal fluoride to afford 2,6-difluorobenzonitrile, and then partially hydrolysing this product to afford the desired 2,6-difluorobenzamide. 2,6-Dichlorobenzonitrile may itself be prepared from 2,6-dichlorotoluene by aminoxidation. This known method suffers from the disadvantage that the final stage partial hydrolysis of the 2,6-difluorobenzonitrile is difficult to perform on a large scale; without great care complete hydrolysis of the nitrile to the acid will ensue. Furthermore the reaction medium is extremely corrosive.

We have now found an advantageous process for the preparation of 2,6-difluorobenzamide which starts from a material directly obtainable by a fluorination reaction, and which affords the amide in a very high yield.

The present invention provides a process for the preparation of 2,6-difluorobenzamide, which comprises reacting 2,6-difluorobenzoyl fluoride, prepared by reacting 2,6-dichlorobenzoyl chloride with an alkali metal fluoride at a temperature in excess of 150°C, with ammonia, in the presence of an inert solvent.

The reaction may for example be carried out in the presence of a polar inert solvent. Suitable solvents include esters, ketones, ethers and nitriles. Mixtures of solvents may be suitable. Preferably a solvent is selected in which the ammonium fluoride formed in the process is insoluble, thus simplifying work-up of the reaction mixture.

Alternatively, the reaction may be carried out in the presence of a two phase aqueous/organic solvent system. In such a system, the organic phase may for example comprise a hydrocarbon or a halogenated hydrocarbon, for example cyclohexane or toluene. It is surprising that such an aqueous/organic system can produce reasonable yields, as it would be expected that the acid fluoride starting material would be hydrolysed rapidly to benzoic acid.

The reaction temperature is preferably in the range of from 0 to 150°C, reflux temperature often being convenient.

The reaction is preferably carried out using excess ammonia, which may be introduced to the reaction system in any convenient manner, for example by bubbling gaseous ammonia through the reaction mixture, by adding liquid ammonia to the reaction mixture, or by using aqueous ammonia solution.

The 2,6-difluorobenzoyl fluoride starting material is prepared by reacting 2,6-dichlorobenzoyl chloride with an alkali metal fluoride at a temperature in excess of 150°C. The reaction temperature is preferably in the range of from 200 to 280°C, especially 220 to 260°C. The reaction is preferably carried out in the presence of an inert solvent, sulphur-containing solvents such as dimethylsulphone and, especially, sulfolane, being preferred.

Any alkali metal fluoride may be used, potassium fluoride being preferred. The molar ratio of alkali metal fluoride to 2,6-difluorobenzoyl chloride is not critical, but preferably excess alkali metal fluoride is used. For example, the molar ratio of alkali metal fluoride to acid chloride is suitably in the range of from 2.2:1 to 7:1, preferably 3:1 to 6:1.

The reaction may be carried out at atmospheric pressure or under excess pressure. Pressures of up to 20, especially up to 10, atmospheres, may be applied if desired or, preferably, the reaction may be carried out in a confined system under autogenous pressure. Alternatively, the reaction may be carried out under atmospheric pressure, and the product distilled off as it is formed.

We have found, surprisingly, that high yields of 2,6-difluorobenzoyl fluoride can be obtained in this way.

2,6-Dichlorobenzoyl chloride is readily synthesised, for example by high-temperature chlorination of 2-chloro-6-nitrotoluene, which is cheap and readily available. Thus the 2-chloro-6-nitrotoluene is reacted with chlorine in the presence of a Lewis acid such as zinc chloride, aluminium chloride or ferric chloride, at a temperature in the range of from 160 to 240°C, preferably 200-220°C, and a pressure of 1.5 to 6 bar, preferably 1.75 to 3 bar. Water is then added gradually while maintaining the temperature and pressure constant, and finally thionyl chloride, until all of the toluene has been converted into 2,6-dichlorobenzoyl chloride.

Thus the present invention provides a very convenient process for the preparation of the desired product in a very small number of steps starting from readily-available starting materials.

The following Examples illustrate the invention.

Example 1. Preparation of 2,6-difluorobenzoyl fluoride.

A 500ml autoclave was charged with sulfolane, (130g), dry potassium fluoride (130g) and 2,6-dichlorobenzoyl chloride (104.8g). The mixture was stirred at 240°C for 16 hours, after which the final pressure reached 6 bars. The mixture was cooled, filtered, and the filtercake washed twice with dichloromethane. The combined filtrates were distilled to remove the solvent, and the residue was distilled at reduced pressure. The largest distillate fraction consisted of 29.6g of the desired product, melting point 35°C. The total yield of the desired product was 41.8g.

Example 2. Preparation of 2,6-difluorobenzoyl fluoride

A 2 liter autoclave was charged with sulfolane (1150g), dry potassium fluoride (369g), and technical 2,6-dichlorobenzoyl chloride, purity 76% (370g). The reactor was flushed with nitrogen, and the stirred mixture was heated to 230°C and kept at that temperature for 17 hours. The reaction mixture was then cooled down, and filtered on a fritted glass filter. The filter cake was washed 3 times with dichloromethane. The combined filtrates were distilled in a batch still equipped with a 10 trays Oldershaw column. The total amount of 2,6-difluorobenzoyl fluoride distilled was 160g (74.6%).

Examples 3 to 5. Preparation of 2,6-difluorobenzamide.

Example 3

2,6-Difluorobenzoylfluoride (16.6g) was dissolved in acetonitrile (100ml) and ammonia gas was bubbled through the stirred solution until no further weight gain occurred. The temperature increased to reflux throughout this addition. The mixture was stirred for a further 15 minutes, then cooled and filtered. The filtrate was distilled, and the solid distillation residue was dried in vacuo overnight to give 16.0g of the desired product as a white solid, melting point 144°C.

Example 4

The method of Example 3 was repeated using acetone as solvent, giving a similar result.

Example 5

2,6-Difluorobenzoylfluoride (10g) was dissolved in cyclohexane (75ml) and concentrated (25%) aqueous ammonia solution (11g) was added over 5 minutes. The mixture was stirred for 1 hour, after which time a further 5g of concentrated aqueous ammonia solution were added. The mixture was heated to 60°C for 1 hour. After cooling, the resultant precipitate was washed and dried to give 8.7g of the desired product. Analysis showed the presence of only 1.5% benzoic acid by-product.

**Claims**

1. A process for the preparation of 2,6-difluorobenzamide, which comprises reacting 2,6-difluorobenzoyl fluoride, prepared by reacting 2,6-dichlorobenzoyl chloride with an alkali metal fluoride at a temperature in excess of 150°C, with ammonia, in the presence of an inert solvent.

2. A process as claimed in claim 1, carried out in the presence of a solvent which is an ester, ketone, ether or nitrile, or in the presence of an aqueous/organic solvent system in which the organic phase comprises a hydrocarbon or halogenated hydrocarbon.

3. A process as claimed in either claim 1 or claim 2, in which the reaction temperature is in the range of from 0 to 150°C.

4. A process as claimed in any of claims 1 to 3, in which 2,6-dichlorobenzoyl chloride has been reacted with an alkali metal fluoride at a temperature in the range of from 200 to 280°C.

5. A process as claimed in any of claims 1 to 4, in which 2,6-dichlorobenzoyl chloride has been reacted with an alkali metal fluoride in the presence of sulfolane as solvent.

6. A process as claimed in any one of claims 1 to 5, in which the molar ratio of alkali metal fluoride to 2,6-dichlorobenzoyl chloride is in the range of from 2.2:1 to 7:1.

**Patentansprüche**

1. Verfahren zur Herstellung von 2,6-Difluorbenzamid, welches ein Umsetzen von 2,6-Difluorbenzoylfluorid, das durch Reagieren von 2,6-Dichlorbenzoychlorid mit einem Alkalimetallfluorid bei einer Temperatur von über 150°C bereitet worden ist, mit Ammoniak in Anwesenheit eines inerten Lösungsmittels umfaßt.

2. Verfahren nach Anspruch 1, das in Anwesenheit eines Lösungsmittels ausgeführt wird, das ein Ester, Keton, Ether oder Nitril ist, oder in Anwesenheit eines wäßrig/organischen Lösungsmittelsystems ausgeführt wird, worin die organische Phase einen Kohlenwasserstoff oder halogenierten Kohlenwasserstoff umfaßt.

3. Verfahren nach Anspruch 1 oder 2, worin die Reaktionstemperatur im Bereich von 0 bis 150°C liegt.

4. Verfahren nach einem der Ansprüche 1 bis 3, worin das 2,6-Dichlorbenzoylchlorid mit eins Alkalimetallfluorid bei einer Temperatur im Bereich von 200 bis 280°C umgesetzt worden ist.

5. Verfahren nach einem der Ansprüche 1 bis 4, worin das 2,6-Dichlorbenzoylchlorid mit einem Alkalimetallfluorid in Anwesenheit von Sulfolan als Lösungsmittel umgesetzt worden ist.

6. Verfahren nach einem der Ansprüche 1 bis 5, worin das Molverhältnis von Alkalimetallfluorid zu 2,6-Dichlorbenzoylchlorid im Bereich von 2,2:1 bis 7:1 liegt.

**Revendications**

1. Un procédé de préparation du 2,6-difluorobenzamide, qui comprend la réaction de fluorure de 2,6-difluorobenzoyle, préparé par réaction de chlorure de 2,6-dichlorobenzoyle avec un fluorure de métal alcalin à une température de plus de 150°C, avec l'ammoniac en présence d'un solvant inerte.

2. Un procédé selon la revendication 1, mis en oeuvre en présence d'un solvant qui est un ester, une cétone, un éther ou un nitrile, ou en présence d'un système de solvants aqueux/organique dans lequel la phase organique comprend un hydrocarbure ou un hydrocarbure halogéné.

3. Un procédé selon la revendication 1 ou la revendication 2, dans lequel la température de réaction est comprise entre 0 et 150°C.

4. Un procédé selon l'une quelconque des revendications 1 à 3, dans lequel le chlorure de 2,6-dichlorobenzoyle a été mis à réagir avec un fluorure de métal alcalin à une température comprise entre 200 et 280°C.

5. Un procédé selon l'une quelconque des revendications 1 à 4, dans lequel le chlorure de 2,6-dichlorobenzoyle a été mis à réagir avec un fluorure de métal alcalin en présence de sulfolane comme solvant.

6. Un procédé selon l'une quelconque des revendications 1 à 5, dans lequel le rapport molaire du fluorure de métal alcalin au chlorure de 2,6-dichlorobenzoyle est compris entre 2,2:1 et 7:1.